# DEMANDE DE BREVET EUROPEEN

(11) **EP 0 639 360 A1**
(43) Date de publication de la demande: **22.02.1995**
(21) Numéro de dépôt: 94401128.7
(22) Date de dépôt: 20.05.1994
(51) Int. Cl.: A61F 2/42

(54) **Prothèse articulaire de doigt avec un élément à bouclier**

(30) Priorité: 28.05.1993 FR 9306452
(71) Demandeur: Lafosse, Laurent, F-74940 Annecy Le Vieux (FR)
(72) Inventeur: Lafosse, Laurent, F-74940 Annecy Le Vieux (FR)
(74) Mandataire: Derambure, Christian

(57) **Abrégé**

L'invention concerne une prothèse articulaire de doigt - notamment métacarpophalangienne ou interphalangienne proximale - comprenant un implant proximal (5a, 5b) associé à un élément osseux proximal (1) ou (2) et formant une surface convexe (7a, 7b) d'articulation et un implant distal (6a, 6b) associé à un élément osseux distal (2) ou (3) formant une surface concave (8a, 8b) d'articulation venant glisser au contact de la surface convexe (7a, 7b) d'articulation, caractérisée en ce que chaque implant est constitué d'un bouclier (9a, 9b, 11a, 11b) dont la forme et les dimensions correspondent au moins sensiblement à celles du cartilage d'origine qui la remplace en recouvrant l'extrémité (17, 18, 19, 20) de l'élément osseux (1, 2, 3) sur ou dans laquelle il est associé par encastrement en préservant les portions extrêmes de l'élément osseux (1, 2, 3) d'ancrage des ligaments (13a, 13b) et/ou des tendons (14a, 14b).

## Description

L'invention concerne une prothèse articulaire de doigt c'est à dire une arthroplastie prothétique - notamment métacarpophalangienne, interphalangienne proximale ou métatarsophalangienne -.

On connaît déjà deux types principaux de prothèses articulaires de doigt. Dans le premier type de prothèse connue, celle-ci est essentiellement constituée d'une pièce d'un seul tenant en matière synthétique telle que le silastic (marque déposée) formant deux cônes opposés introduits de chaque côté dans les éléments osseux à relier, les deux cônes étant liés par une partie souple. On constate néanmoins avec une telle prothèse que l'amplitude angulaire de flexion des éléments osseux l'un par rapport à l'autre est relativement faible en pratique. De plus, du fait de son encombrement longitudinal et de son mode d'utilisation, la pose d'une telle prothèse rend difficile voire impossible la conservation du manchon capsuloligamentaire et de l'appareil tendineux de l'articulation d'origine. Dès lors, l'articulation réalisée par la prothèse comporte une certaine instabilité latérale. Egalement, on constate souvent un phénomène d'ostéolyse au contact des éléments osseux avec les cônes de la prothèse, qui engendre un raccourcissement du doigt et une détente de l'appareil ligamentaire et tendineux éventuellement conservé. Par ailleurs, une telle prothèse peut engendrer dans certains cas des phénomènes d'allergie. De plus, la matière synthétique utilisée, soumise à des contraintes en flexion répétées, peut fatiguer et subir un phénomène de vieillissement prématuré. Enfin, une telle prothèse suppose la suppression d'une grande partie de l'articulation d'origine sans pour autant remplacer l'intégralité des fonctions mécaniques de cette articulation d'origine. Par exemple les possibilités d'écartement latéral des doigts par rapport aux métacarpiens sont supprimées ou largement diminuées.

Le deuxième type de prothèse connue (notamment des documents FR-A-2 605 878, US-A-4 725 280, WO-A-89 06946, US-A-5 037 440), dite à glissement, comporte un implant mâle constitué d'un condyle en forme généralement de globe prolongé ou non par un cône de grande largeur à redans inséré dans l'élément osseux, et un implant femelle formant une surface complémentaire au condyle et prolongé ou non par un cône à redans inséré dans l'autre élément osseux. Là encore, compte tenu de l'encombrement axial et transversal du condyle et de l'implant femelle, l'articulation d'origine doit souvant être supprimée. Pour assurer la stabilité latérale, on prévoit une gorge dans la surface complémentaire de l'implant femelle et une nervure en saillie axiale par rapport au condyle coopérant avec la gorge. Ce deuxième type de prothèse connue suppose donc l'insertion de cônes d'encombrement transversal sensiblement important dans chaque élément osseux, ce qui engendre la suppression ou l'excision d'une trop grande partie du canal médullaire et donc une atteinte importante aux caractéristiques biologiques de chaque élément osseux. De plus, la pose d'une telle prothèse suppose là encore le sacrifice de l'intégralité de l'appareil ligamentaire de l'articulation et empêche toute réutilisation des tendons d'origine. Egalement, ces prothèses engendrent des phénomènes d'ostéolyse importants dus notamment à l'atteinte par les cônes de la partie corticale de chaque élément osseux. Et là encore, les possibilités d'écartement latéral des doigts en extension par rapport aux métacarpiens sont supprimées.

Or, on constate en pratique que les affections subies au niveau d'une articulation de doigt peuvent être classées en trois catégories de gravité croissante :
- les affections dans lesquelles seules les parties cartilagineuses de l'articulation sont atteintes, l'appareil ligamentaire et tendineux étant préservé. Tel est particulièrement le cas des arthroses ou des affections dues à des séquelles d'entorse, de fracture ...
- les affections dans lesquelles les cartilages et l'appareil ligamentaire sont atteints mais où les éléments osseux sont préservés, telles que par exemple les arthrites...
- les affections dans lesquelles l'intégralité de l'articulation est atteinte, y compris les éléments osseux telles que les arthrites évoluées ou les affections de type traumatique dues par exemple à des accidents.

Or, on constate que les deux types de prothèses connues susmentionnées procurent, dans le cas de la première catégorie d'affection, une solution similaire à celle procurée dans les deux autres catégories, c'est à dire en supprimant l'intégralité de l'articulation, y compris les ligaments et les tendons.

L'invention a donc pour objet de pallier les inconvénients susmentionnés des prothèses connues.

Plus particulièrement, l'invention a pour objet de proposer une prothèse articulaire de doigt qui peut être posée en préservant l'appareil ligamentaire et/ou tendineux de l'articulation et avec une atteinte réduite du manchon capsulo-ligamentaire. L'invention a également pour objet de proposer une telle prothèse qui calque au plus près l'anatomie d'origine de l'articulation, en respectant les organes de l'articulation non atteints par l'affection en fonction de sa gravité.

L'invention a également pour objet de proposer une prothèse articulaire de doigt dont la pose n'engendre que peu de traumatisme dans chacun des éléments osseux à relier.

L'invention a également pour objet de proposer une prothèse articulaire de doigt qui permet de résoudre l'intégralité des affections rencontrées quel que soit leur degré de gravité, et qui puisse être adaptée immédiatement lors de l'opération chirurgicale en fonction des besoins selon que les éléments osseux et/ou ligamentaires et/ou tendineux sont atteints.

L'invention a également pour objet de proposer une prothèse articulaire de doigt avec laquelle les risques d'allergie ou d'ostéolyse sont diminués.

L'invention a également pour objet de proposer une prothèse articulaire de doigt dont les caractéristiques mécaniques sont améliorées et se rapprochent le plus possible de l'anatomie originelle, plus particulièrement dont l'encombrement longitudinal soit plus faible que dans l'art antérieur et qui préserve les possibilités d'écartement des doigts par rapport aux métacarpiens, dans le cas d'une prothèse métacarpophalangienne.

L'invention a également pour objet de proposer une prothèse articulaire de doigt comprenant des éléments constitutifs simples, de faible prix de revient, qui sont semblables quel que soit le doigt ou le métacarpien considéré et dont la pose est extrêmement simple et rapide.

Plus particulièrement, l'invention a pour objet de proposer une prothèse articulaire de doigt qui peut être posée en préservant l'appareil ligamentaire de l'articulation ou en permettant sa reconstruction par des ligaments artificiels. Egalement, l'invention a pour objet de proposer une prothèse articulaire de doigt qui permette de réutiliser les tendons naturels. Egalement l'invention a pour objet de proposer une telle prothèse qui soit adaptable dans le cas où les éléments osseux sont atteints, et dont la longueur des implants peut être adaptée selon les besoins.

Pour ce faire, l'invention concerne une prothèse articulaire de doigt - notamment métacarpophalangienne ou interphalangienne proximale - comprenant un implant proximal associé à un élément osseux proximal et formant une surface convexe d'articulation, et un implant distal associé à un élément osseux distal et formant une surface concave d'articulation venant glisser au contact de la surface convexe d'articulation, caractérisée en ce que chaque implant comprend un bouclier dont la forme et les dimensions correspondent au moins sensiblement à celles du cartilage d'origine qu'il remplace en recouvrant l'extrémité de l'élément osseux sur ou dans laquelle il est associé par encastrement en préservant les portions extrêmes de l'élément osseux d'ancrage des ligaments et/ou des tendons. Selon l'invention, la prothèse est caractérisée en ce que chaque bouclier recouvre l'extrémité de l'élément osseux en laissant libres au moins les parties latérales de cette extrémité. Selon l'invention, la prothèse est caractérisée en ce qu'un implant comprend un prolongement d'ancrage s'étendant dans une partie évidée de l'élément osseux. Ce prolongement d'ancrage est fin et orienté pour optimiser la position du bouclier par rapport à l'os.

Selon l'invention, les surfaces de contact de l'un des implants - notamment de l'implant proximal - avec l'élément osseux correspondant sont telles qu'elles autorisent son encastrement par insertion de cet implant selon une direction parallèle ou confondue à l'axe principal longitudinal de cet élément osseux. Et les surfaces de contact de l'autre implant - notamment de l'implant distal - avec l'élément osseux correspondant sont telles qu'elles autorisent son encastrement par insertion de cet implant selon une direction transversale à l'axe principal longitudinal de l'élément osseux.

Selon l'invention, l'implant proximal est constitué d'un bouclier proximal qui entoure l'extrémité de l'élément osseux encastrée à l'intérieur d'une cavité formée par ce bouclier proximal. Le bouclier proximal est en forme générale de coquille formant au moins sensiblement une portion de sphère ou de tore ou de double sphère ou de double tore. Ainsi, l'invention concerne en particulier une prothèse métacarpophalangienne ou métatarsophalangienne caractérisée en ce que le bouclier proximal définit une surface convexe qui présente une courbure autour d'un axe de courbure vertical - notamment en forme de portion de sphère ou de portion de tore - pour permettre notamment l'écartement des doigts en extension. Et le prolongement d'ancrage de l'implant proximal est une tige s'étendant axialement dans un perçage longitudinal de la partie médullaire de l'élément osseux correspondant. Selon l'invention, la tige s'étend à partir de la surface interne du bouclier décalée vers le haut par rapport à l'axe médian longitudinal du bouclier. Dans le cas d'une prothèse interphalangienne, l'implant proximal est pourvu ou non de prolongement d'ancrage et est constitué d'un bouclier proximal.

Selon l'invention, l'implant distal est constitué d'un bouclier distal encastré dans un évidement ménagé dans l'extrémité de l'élément osseux qui entoure ce bouclier distal. Et le bouclier distal est globalement en forme de plaque transversale dont la surface de contact avec l'élément osseux est plane et dont la surface libre forme la surface concave d'articulation. Le prolongement d'ancrage de l'implant distal est globalement en forme d'aileron ou de rail s'étendant dans un plan longitudinal médian de l'élément osseux au moins sensiblement perpendiculaire à l'axe principal de pivotement de l'articulation.

Selon l'invention, au moins un prolongement d'ancrage comporte au moins un perçage transversal permettant sa fixation sur l'élément osseux par une cheville ou une vis transversale traversant l'élément osseux ; au moins un perçage transversal permettant la fixation d'un ligament naturel ou artificiel à ce prolongement d'ancrage, notamment par une cheville ou une vis coinçant ce ligament dans le perçage transversal ; au moins un perçage transversal de fixation d'un tendon.

Ainsi, les boucliers des implants d'une prothèse selon l'invention ont des formes qui permettent leur encastrement à l'extrémité de chaque élément osseux. Et les prolongements d'ancrage peuvent être ainsi de dimension réduite, notamment en ce qui concerne leur encombrement transversal. Dès lors, les prolongements d'ancrage sont introduits dans le canal médullaire de l'élément osseux sur une faible longueur et une faible épaisseur et préservent non seulement les parties corticales, mais également la majeure partie de la portion médullaire de l'élément osseux.

De plus, chaque implant peut être posé aisément sans destruction de l'articulation d'origine, notamment de l'appareil ligamentaire et/ou de l'appareil tendineux. Par exemple, l'implant proximal est posé en fléchissant l'élément osseux distal à 90° en position extrême, et en insérant la tige formant le prolongement d'ancrage axialement entre les ligaments. Puis l'implant distal est inséré transversalement par dessus entre les ligaments. La majeure partie de la matière osseuse est préservée et les phénomènes d'ostéolyse sont évités. Ainsi, les caractéristiques mécaniques naturelles de l'articulation sont préservées au maximum. De plus, dans le cas où l'articulation est atteinte de façon plus sévère, l'appareil ligamenteux peut être reconstruit à l'aide de ligaments artificiels posés de façon exactement similaire à l'appareil ligamentaire naturel. Dans le cas où une portion sensiblement importante d'un os est atteinte, l'implant correspondant peut être posé sur un manchon de prolongation artificiel interposé entre l'extrémité de l'os et l'implant correspondant.

L'invention concerne également une prothèse articulaire de doigt comportant en combinaison tout ou partie des caractéristiques mentionnées ci-dessus ou ci-après.

D'autres caractéristiques et avantages de l'invention apparaîtront à la lecture de la description qui se réfère aux figures annexées représentant un mode de réalisation préférentiel et, dans lesquelles :
- la figure 1 est une vue schématique en section axiale d'un doigt de main comportant une prothèse articulaire selon l'invention sur son articulation métacarpophalangienne, et une prothèse selon l'invention sur son articulation interphalangienne proximale.
- la figure 2 est une vue de dessus de la figure 1.
- la figure 3 est une vue similaire à la figure 1 illustrant des variantes de réalisation des prothèses selon l'invention.
- les figures 4 à 7 sont des vues de profil, de gauche, de dessus et en perspective d'un implant proximal d'une prothèse d'articulation interphalangienne proximale selon l'invention.
- les figures 8 à 10 sont des vues de profil, de droite, et en coupe selon la ligne A/A de la figure 8 d'un implant distal d'une prothèse d'articulation interphalangienne proximale selon l'invention.
- les figures 11 et 12 sont des vues similaires à la figure 8 illustrant des variantes de réalisation d'un implant distal selon l'invention.

L'invention concerne une prothèse articulaire de doigt destinée à relier de façon articulée un élément osseux proximal 1 ou 2 à un élément osseux distal 2 ou 3.

Sur les figures, on a représenté une prothèse articulaire métacarpophalangienne 4a entre un métacarpien 1 formant l'élément osseux proximal et la première phalange 2 (ou phalange proximale) formant l'élément osseux distal pour cette prothèse 4a. On a également représenté une prothèse selon l'invention interphalangienne proximale 4b interposée entre la première phalange 2 formant l'élément osseux proximal 2 de cette prothèse 4b et une phalangine 3 formant l'élément osseux distal 3 pour cette prothèse 4b. Ainsi, dans tout le texte, les adjectifs "proximal" et "distal" sont utilisés pour exprimer les positions relatives par rapport à la prothèse articulaire selon l'invention considérée 4a ou 4b. De même, les lettres "a" dans les références numériques se réfèrent aux éléments constitutifs de la prothèse métacarpophalangienne 4a et les lettres "b" dans les références numériques se réfèrent aux éléments constitutifs de la prothèse interphalangienne proximale 4b.

Une prothèse articulaire selon l'invention comprend essentiellement deux pièces d'un seul tenant chacune, à savoir un implant proximal 5a, 5b associé à un élément osseux proximal 1 ou 2 et un implant distal 6a, 6b associé à un élément osseux distal 2 ou 3. Et l'implant proximal 5a, 5b forme une surface convexe 7a, 7b d'articulation et l'implant distal 6a, 6b forme une surface concave 8a, 8b d'articulation qui vient glisser au contact de la surface convexe 7a, 7b d'articulation.

Selon l'invention, chaque implant 5a, 5b, 6a, 6b comporte un bouclier 9a, 9b, 11a, 11b dont la forme et les dimensions correspondent au moins sensiblement à celles du cartilage naturel d'origine qu'il remplace en recouvrant l'extrémité 17, 18, 19, 20 de l'élément osseux 1, 2, 3 sur ou dans laquelle il est associé par encastrement en préservant les portions extrêmes de l'élément osseux 1, 2, 3 servant à l'ancrage des ligaments 13a, 13b et/ou des tendons 14a, 14b. Chaque bouclier 9a, 9b, 11a, 11b forme ainsi un revêtement rigide de faible épaisseur de l'extrémité de l'élément osseux 1, 2, 3 dont les portions extrêmes sont conservées ou remplacées. Selon l'invention un implant 5a, 5b, 6a, 6b comporte en outre un prolongement d'ancrage 10a, 10b, 12a, 12b s'étendant dans une partie évidée axialement de l'élément osseux 1, 2, 3. Selon l'invention, les parties latérales de chaque bouclier 9a, 9b, proximal sont ouvertes de façon que les parties latérales de l'extrémité 17, 19 de l'élément osseux 1, 2 soient libres et accessibles, l'ancrage des ligaments étant préservé ou pouvant être reconstitué.

Selon l'invention, les surfaces de contact 15, 16 de l'un 5b des implants - notamment de l'implant proximal 5b - avec l'élément osseux correspondant sont telles qu'elles autorisent l'encastrement de l'implant proximal 5b par insertion de cet implant 5b selon une direction parallèle ou confondue à l'axe longitudinal de cet élément osseux 2. Et les surfaces de contact 21, 22 de l'autre 6b implant - notamment de l'implant distal 6b - avec l'élément osseux 3 correspondant sont telles qu'elles autorisent son encastrement par insertion de cet implant 6b selon une direction transversale à l'axe longitudinal de cet élément osseux 3. Cela est vrai pour la prothèse articulaire métacarpophalangienne 4a comme pour la prothèse articulaire interphalangienne 4b.

Selon l'invention, chaque prolongement d'ancrage 10a, 10b, 12a, 12b est formé d'un seul tenant avec au moins une portion du bouclier 9a, 9b, 11a, 11b de l'implant 5a, 5b, 6a, 6b.

Selon l'invention, l'implant proximal 5a, 5b comporte un bouclier proximal 9a, 9b qui entoure l'extrémité 17, 19 distale de l'élément osseux 1, 2, proximal, cette extrémité 17, 19 étant encastrée à l'intérieur d'une cavité 23a, 23b formée par ce bouclier proximal 9a, 9b. Le bouclier 9a, 9b proximal est en forme de calotte ou de coquille dont la surface extérieure 7a, 7b est convexe et constitue la surface convexe d'articulation. La surface interne 15 du bouclier proximal 9a, 9b constitue la surface délimitant la cavité 23a, 23b.

Selon l'invention, le bouclier 9a de l'implant proximal 5a d'une prothèse métacarpophalangienne 4a (ou métatarsophalangienne) définit une surface convexe 7a externe qui présente une première courbure autour d'un axe de courbure horizontal pour permettre la flexion de la première phalange 2 par rapport au métacarpien 1 autour d'un axe de pivotement 26a principal horizontal, mais également une seconde courbure autour d'un axe de courbure vertical pour permettre l'écartement des doigts en extension. Le bouclier 9a proximal de la prothèse métacarpophalangienne 4a est ainsi en forme de portion de sphère ou de portion de tore. Cette forme sphérique permet ainsi de préserver les possibilités d'écartement des doigts, et de compenser l'écartement en éventail des métacarpiens 1 en n'utilisant qu'un seul modèle d'implant pour tous les métacarpiens.

Selon l'invention et plus particulièrement pour le bouclier proximal 9a de la prothèse métacarpophalangienne 4a, la cavité 23a est sphérique ou torique comme la surface extérieure 7a convexe de ce bouclier 9a. Et la surface de contact de l'extrémité distale 17 du métacarpien est conformée en portion de sphère ou de tore pour permettre son encastrement dans la cavité sphérique ou torique 23a.

Selon l'invention, et plus particulièrement pour le bouclier proximal 9b de la prothèse interphalangienne proximale 4b, la cavité 23b est polyédrique, c'est à dire que la surface interne 15 du bouclier 9b est constituée de plans venant au contact de surfaces planes correspondantes de l'extrémité 19 de l'élément osseux 1, 2 correspondant. La cavité 23b est alors formée d'une gorge d'axe parallèle à l'axe 26a, 26b principal de pivotement de l'articulation et dont la section droite est en forme générale de U. De la sorte les surfaces de contact de l'extrémité 19 distale avec le bouclier comportent essentiellement des plans parallèles à l'axe 26b de pivotement, à savoir deux plans longitudinaux supérieur ou inférieur parallèles à l'axe longitudinal de l'élément osseux 2 et un plan d'extrémité perpendiculaire à l'axe longitudinal de l'élément osseux 2 et reliant les plans longitudinaux supérieur et inférieur.

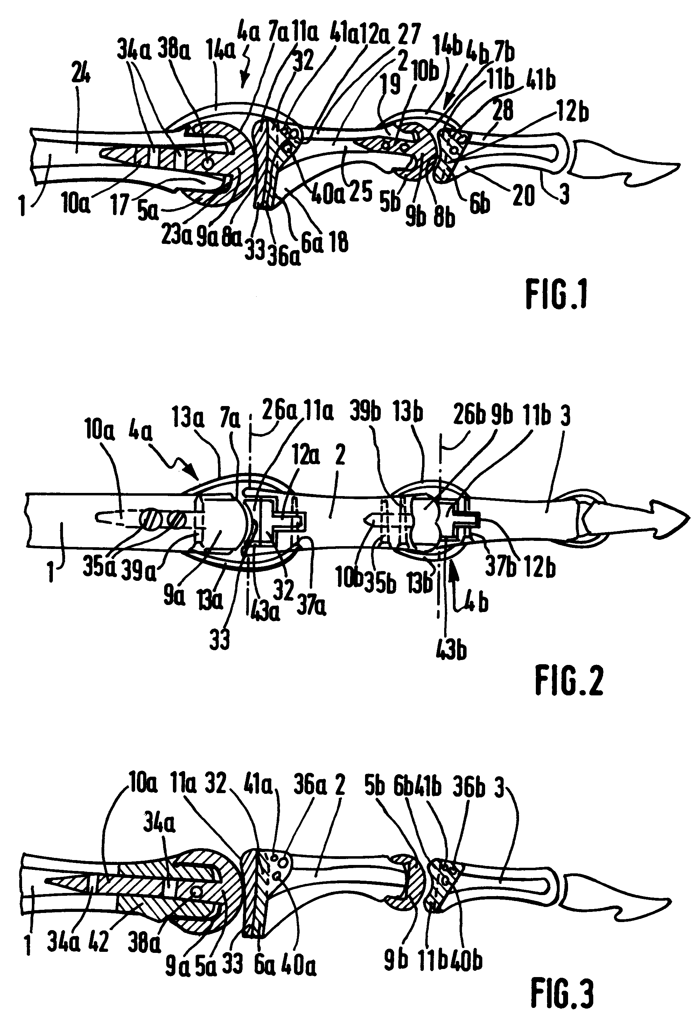

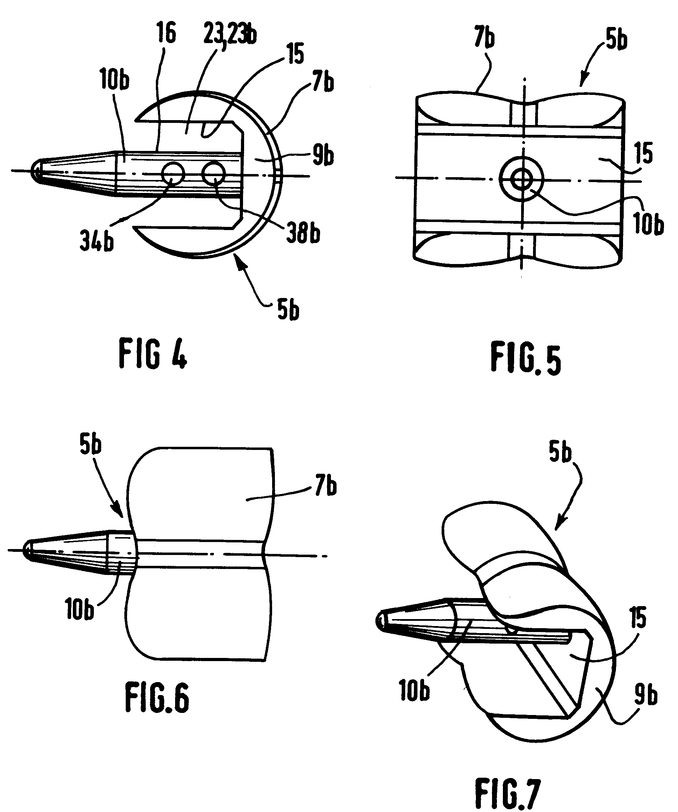

Selon l'invention, le prolongement d'ancrage 10a, 10b de l'implant proximal 5a, 5b est une tige 10a, 10b s'étendant axialement dans un perçage longitudinal de la partie médullaire 24, 25 de l'élément osseux 1, 2 correspondant. La longueur de la tige 10a, 10b varie en fonction des dimensions de l'élément osseux 1, 2, de l'implant proximal 5a, 5b et de l'état général de l'articulation naturelle et de ses éléments fonctionnels. La largeur de la tige 10a, 10b est normalement plus faible que la largeur de la partie médullaire 24, 25 de l'élément osseux 1, 2 à son extrémité distale 17, 19 recevant la tige 10a, 10b. La tige 10a, 10b est terminée par une extrémité pointue pour faciliter son introduction. La position et l'orientation de la tige 10a, 10b sont déterminées pour permettre une mise en place automatique du bouclier par rapport à l'axe du canal médullaire et en respectant les contraintes cinématiques et anatomiques. La tige 10a, 10b a des dimensions radiales inférieures à celles du canal médullaire 24, 25 de l'os dans lequel elle doit être insérée. Sa longueur peut varier selon les besoins, c'est à dire selon la gravité de l'affection rencontrée de façon qu'elle pénètre sur une distance plus ou moins importante dans l'élément osseux. L'implant proximal 5b de la prothèse articulaire interphalangienne proximale 4b peut même être dépourvu de tige d'ancrage et n'être constitué que du bouclier 9b enchâssé à l'extrémité distale 19 de la première phalange 2 (variante représentée figure 3). Dans le cas où une telle tige 10b est prévue sur la prothèse interphalangienne proximale 4b, celle-ci s'étend perpendiculairement à l'âme de la cavité 23b en forme de U et à mi-distance de ses ailes (figure 4).

Dans le cas de la prothèse métacarpophalangienne 4a, la tige 10a est décalée vers le haut par rapport à l'axe médian horizontal longitudinal de symétrie du bouclier 9a proximal, ce qui permet d'incliner la tige 10a par rapport à l'orientation de l'axe médullaire à l'extrémité de l'os et de tenir compte de la dissymétrie axiale du métacarpien 1. Egalement cette position décalée permet d'avoir une amplitude de flexion de la première phalange 2 par rapport au métacarpien 1 supérieure à 95°, notamment de l'ordre de 100°, et de bloquer automatiquement le bouclier 9a en rotation par rapport à l'axe médian longitudinal de symétrie de l'extrémité 17 de l'élément osseux 1.

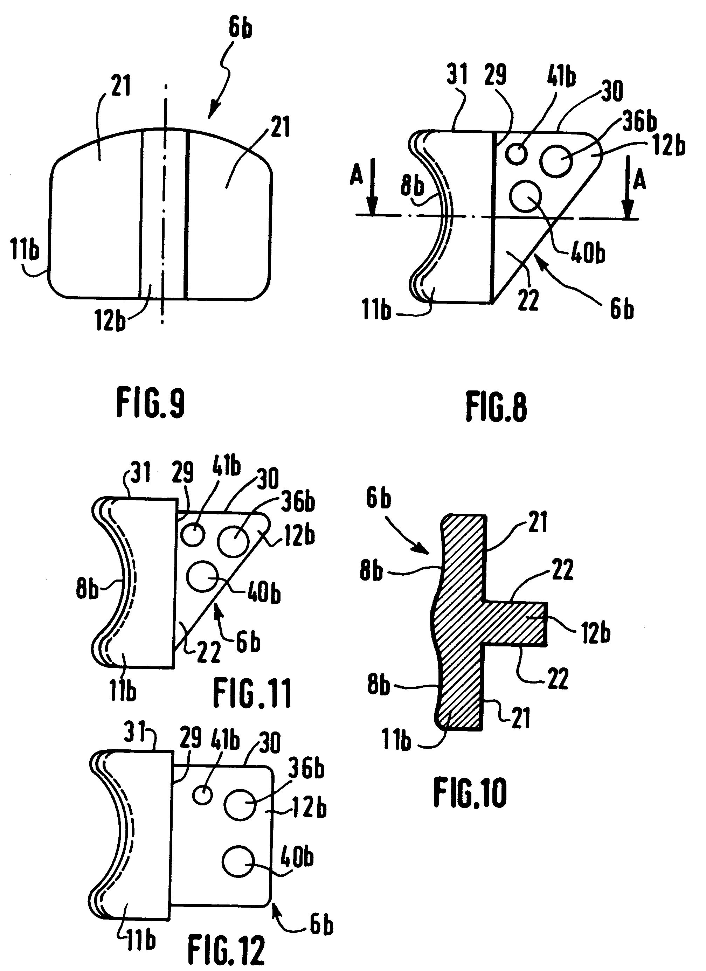

Selon l'invention, l'implant distal 6a, 6b comporte un bouclier distal 11a, 11b encastré dans un évidement ménagé dans l'extrémité proximale 18, 20 de l'élément osseux 2, 3 correspondant. Cet évidement entoure le bouclier distal 11a, 11b qui est encastré dans l'extrémité 18, 20 de l'élément osseux 2, 3. Cet évidement est ménagé entre les parties latérales 43a, 43b de l'extrémité 18, 20 qui sont préservées et enserrent le bouclier distal 11a, 11b. Selon l'invention, le bouclier distal 11a, 11b est globalement en forme de plaque globalement plane transversale dont la surface de contact 21 transversale avec l'élément osseux 2, 3 est plane et dont la surface libre forme la surface concave 8a, 8b d'articulation. Et le prolongement d'ancrage 12a, 12b de l'implant distal 6a, 6b est en forme d'aileron 12a, 12b s'étendant dans un plan longitudinal médian de l'élément osseux 2, 3 au moins sensiblement perpendiculaire à l'axe principal 26a, 26b de pivotement de l'articulation horizontale perpendiculaire à l'axe longitudinal du doigt. Selon l'invention, la majeure partie de l'aileron 12a, 12b d'ancrage de l'implant distal 6a, 6b s'étend dans une partie évidée de la portion corticale supérieure 27, 28 de l'élément osseux 2, 3. Et l'aileron 12a, 12b est en forme de triangle rectangle dont un côté 29, adjacent au bouclier distal (11a, 11b), s'étend sur au moins une partie de la hauteur médiane du bouclier distal 11a, 11b et dont l'autre côté 30 perpendiculaire s'étend longitudinalement à partir du bord extrême supérieur 31 du bouclier distal 11a, 11b vers l'extrémité du doigt (variante représentée figure 8). Cette forme triangulaire permet, avec une forme triangulaire correspondante de la partie évidée de l'élément osseux 2, 3, de former une butée d'arrêt du bouclier 11a, 11b en fin d'insertion dans la direction transversale. Comme illustré sur la figure 11, le côté 30 du triangle rectangle peut ne pas s'étendre à partir du bord extrême supérieur 31 mais décalé par rapport à celui-ci.

Dans une autre variante (figure 12), l'implant distal 6a, 6b est en forme de rail 12a, 12b et non d'aileron.

Le bouclier proximal 9a, 9b est métallique, susceptible d'être réhabitable par l'élément osseux, et le bouclier distal 11a, 11b est au moins pour sa portion formant la surface concave 8a, 8b d'articulation, en matière synthétique - notamment en polyéthylène - ou en céramique. Selon l'invention, l'implant proximal 5a, 5b est constitué d'une pièce métallique monobloc, également susceptible d'être réhabitable par l'élément osseux ou d'une pièce en céramique monobloc. Dans le cas de la prothèse articulaire interphalangienne proximale, l'implant distal 6b est constitué d'une pièce monobloc en matière synthétique - notamment en polyéthylène -. Dans le cas de la prothèse articulaire 4a métacarpophalangienne, l'implant distal 6a est constitué de deux pièces associées l'une à l'autre : une pièce métallique ou en céramique 32 interne, susceptible d'être réhabitable par l'élément osseux, formant le prolongement d'ancrage 12a en forme d'aileron et la partie interne du bouclier 9a, et une pièce de matière synthétique 33 - notamment en polyéthylène - ou en céramique formant la partie externe du bouclier 9a et recouvrant la partie interne métallique ou en céramique. La pièce de matière synthétique 33 est rapportée par exemple par encliquetage ou par collage sur la pièce métallique ou en céramique 32. Ainsi, un implant 5a, 5b, 6a, 6b proximal ou distal d'une prothèse selon l'invention est soit entièrement métallique, soit en matière synthétique, soit composite, c'est à dire composée d'une portion métallique et d'une portion en matière synthétique.

Par ailleurs, dans une prothèse selon l'invention, au moins un prolongement d'ancrage 10a, 10b, 12a, 12b comporte au moins un perçage transversal 34a, 34b, 36a, 36b permettant la fixation de l'implant 5a, 5b, 6a, 6b correspondant par une cheville ou une vis 35a, 35b, 37a, 37b transversale traversant l'élément osseux 1, 2, 3 et le perçage 34a, 34b, 36a, 36b. Si nécessaire, on peut prévoir plusieurs perçages transversaux 34a, 34b, 36a, 36b et plusieurs vis ou chevilles 35a, 35b, 37a, 37b pour fixer l'implant 5a, 5b, 6a, 6b à l'élément osseux 1, 2, 3 également percé. Dans le cas d'une prothèse métacarpophalangienne 4a, les perçages 34a de l'implant proximal 5a peuvent s'étendre verticalement pour faciliter la pose de vis ou chevilles à travers le métacarpien 1. Dans les autres cas, les perçages 34b, 36a, 36b peuvent s'étendre horizontalement.

Par ailleurs, au moins un prolongement d'ancrage 10a, 10b, 12a, 12b comporte au moins un perçage transversal 38a, 38b, 40a, 40b permettant la fixation d'un ligament naturel ou artificiel 13a, 13b à ce prolongement d'ancrage 10a, 10b, 12a, 12b, notamment par une cheville ou une vis 39a, 39b, coinçant ce ligament 13a, 13b dans le perçage transversal 38a, 38b, 40a, 40b. Un tel perçage transversal 38a, 38b, 40a, 40b s'étend horizontalement et en regard de l'extrémité libre 17, 18, 19, 20 de l'élément osseux 1, 2, 3 dont les parties latérales sont aussi percées.

Et la prothèse selon l'invention comporte au moins un prolongement d'ancrage 12a, 12b qui comporte au moins un perçage transversal 41a, 41b de fixation d'un tendon 14a, 14b. En particulier, le tendon 14a, 14b peut être suturé par un fil passant dans le perçage 41a, 41b.

Comme cela apparaît sur les figures 1 à 3, les ligaments 13a, 13b, naturels ou artificiels, s'étendent de chaque côté latéralement des articulations et des prothèses articulaires 4a, 4b. Au contraire, les tendons 14a, 14b s'étendent au dessus des articulations et des prothèses articulaires 4a, 4b. Les perçages d'ancrage 41a, 41b des tendons 14a, 14b sont prévus plus particulièrement sur les ailerons 12a, 12b des implants distaux 6a, 6b, puisque les parties médianes supérieures des portions corticales des éléments osseux 2, 3 correspondants sont évidées pour le passage de l'aileron 12a, 12b, ce qui nécessite généralement la déconnexion du tendon 14a, 14b de l'élément osseux 2, 3.

Sur la figure 3, on a représenté une variante de réalisation de la prothèse articulaire métacarpophalangienne 4a qui comporte un manchon 42 de prolongation artificiel d'élément osseux interposé entre l'extrémité distale 17 d'au moins un os 1, c'est à dire du métacarpien 1, et l'implant 5a proximal correspondant. Un tel manchon artificiel est connu en lui-même et peut être prévu à chaque extrémité proximale ou distale de chaque os relié par une prothèse selon l'invention. L'extrémité du manchon 42 supportant l'implant correspondant est profilée comme l'extrémité libre de l'élément osseux dans la variante précédente de façon à former les surfaces planes de contact ou les évidements permettant l'encastrement de l'implant. Dans tout le texte, l'expression "élément osseux" désigne donc soit un os du doigt, soit un manchon artificiel remplaçant un tel os, soit un élément composite formé d'une portion d'os naturel et d'une portion d'os artificiel.

Dans le mode de réalisation représenté et selon l'invention, le prolongement d'ancrage 10a de l'implant 5a est prolongé longitudinalement pour traverser axialement tout le manchon 42 pour être associé rigidement à l'extrémité 17 distale de l'os 1 grâce à un perçage transversal 34a et à une vis 35a traversant l'os 1 comme décrit précédemment. En variante, on peut associer rigidement le prolongement d'ancrage de l'implant directement au manchon 42, et relier le manchon 42 à l'os 1 par une plaquette de fixation comprenant des perçages à l'aide de vis ou de chevilles. On peut également remplacer l'intégralité d'un des os 1, 2, 3 par un tel manchon 42 artificiel.

Sur les figures 4 à 7, on a représenté un exemple de réalisation d'un implant proximal 5b d'une prothèse articulaire interphalangienne proximale 4b, dont le bouclier 9b est en forme de coquille dont la surface convexe externe 7b est en forme de deux portions de sphères ou de tores semblables accolées se jouxtant dans la direction latérale horizontale. Cette forme procure une stabilité latérale à l'articulation qui n'est mobile qu'en flexion et en extension autour de l'axe 26b de pivotement horizontal.

Sur les figures 8 à 10, on a représenté l'implant distal 6b correspondant en matière synthétique formant la surface concave 8b venant coopérer par contact et glissement avec la surface convexe 7b de l'implant proximal 5b. L'aileron triangulaire 12b constituant le prolongement d'ancrage de cet implant distal 6b comporte trois perçages à savoir un perçage transversal 34b pour la fixation de l'implant par rapport à l'élément osseux 3, un perçage 40b pour la fixation d'un ligament 13b, et un perçage 41b pour la fixation d'un tendon 14b.

Pour poser une prothèse selon l'invention, il suffit de réaliser aux extrémités libres des éléments osseux correspondants, du côté proximal, des surfaces de forme complémentaire aux surfaces internes 15 de la cavité 23 de l'implant proximal, et du côté distal, des évidements dans l'élément osseux distal 2 ou 3 permettant la réception de l'implant distal. On fléchit ensuite l'élément osseux distal 2, 3 à 90° par rapport à l'élément osseux proximal 1 ou 2 et on insère axialement l'implant proximal en introduisant la tige 10a, 10b dans la partie médullaire de l'extrémité distale 17, 19 de cet élément osseux 1 ou 2, et ce entre les ligaments 13a, 13b qui s'étendent de chaque côté de l'articulation. On insère ensuite entre les ligaments 13a, 13b transversalement l'implant distal 6a, 6b jusqu'à ce qu'il vienne en butée, l'aileron 12a, 12b étant complètement inséré dans l'extrémité proximale 18, 20 de l'élément osseux distal 2 ou 3. On suture ensuite à nouveau éventuellement le tendon 14a, 14b à l'extrémité proximale 18, 20 de l'élément osseux distal 2 ou 3. On insère ensuite les vis ou chevilles permettant l'association rigide des implants et le coincement des ligaments dans les perçages des prolongements d'ancrage.

Grâce à l'invention, on préserve les parties extrêmes latérales de chaque élément osseux sur lesquelles les ligaments naturels ou artificiels sont ancrés. De plus la pose de la prothèse peut être réalisée avec une ouverture de faible dimension ménagée dans le manchon capsuloligamentaire.

L'invention a été décrite en référence à des prothèses d'articulation métacarpophalangienne et interphalangienne proximale d'un doigt de la main, mais est applicable à d'autres articulations du même type dans lesquelles les mêmes problèmes se posent, par exemple aux articulations métatarsophalangiennes ou interphalangiennes distales, notamment du pouce.

La matériel ancillaire spécifique de pose d'une prothèse selon l'invention se compose notamment :
- d'un couteau cylindrique à fond sphérique associé de façon amovible en saillie interne dans une cloche pour préparer l'extrémité 17 distale d'un métacarpien en forme de sphère ou de tore en tournant ce couteau autour de l'extrémité
- d'un double ciseau droit pour la préparation des plans longitudinaux horizontaux de l'extrémité distale 19 de phalange à insérer dans une cavité en U en frappant axialement sur ce double ciseau
- d'une cloche comprenant un orifice formant guide de perçage pour le perçage ménagé dans le canal médullaire à l'extrémité distale 17, 19 pour l'introduction d'une tige d'ancrage 10a, 10b.

## Revendications

1. Prothèse articulaire de doigt - notamment métacarpophalangienne ou interphalangienne proximale - comprenant un implant proximal (5a, 5b) associé à un élément osseux proximal (1) ou (2) et formant une surface convexe (7a, 7b) d'articulation et un implant distal (6a, 6b) associé à un élément osseux distal (2) ou (3) formant une surface concave (8a, 8b) d'articulation venant glisser au contact de la surface convexe (7a, 7b) d'articulation caractérisée en ce que chaque implant (5a, 5b, 6a, 6b) comprend un bouclier (9a, 9b, 11a, 11b), dont la forme et les dimensions correspondent au moins sensiblement à celles du cartilage d'origine qu'il remplace, en recouvrant l'extrémité (17, 18, 19, 20) de l'élément osseux (1, 2, 3) sur ou dans laquelle il est associé par encastrement en préservant les portions extrêmes de l'élément osseux (1, 2, 3) d'ancrage des ligaments (13a, 13b) et/ou des tendons (14a, 14b).

2. Prothèse selon la revendication 1 caractérisée en ce que chaque bouclier (9a, 9b, 11a, 11b) recouvre l'extrémité (17, 18, 19, 20) de l'élément osseux en laissant libres au moins les parties latérales de cette extrémité (17, 18, 19, 20).

3. Prothèse selon l'une des revendications 1 et 2 caractérisée en ce qu'un implant (5a, 5b, 6a, 6b) comprend un prolongement d'ancrage (10a, 10b, 12a, 12b) s'étendant dans une partie évidée de l'élément osseux (1, 2, 3).

4. Prothèse selon la revendication 3 caractérisée en ce que chaque prolongement d'ancrage (10a, 10b, 12a, 12b) est formé d'un seul tenant avec au moins une portion du bouclier (9a, 9b, 11a, 11b).

5. Prothèse selon l'une des revendications 1 à 4 caractérisée en ce que les surfaces de contact (15, 16) de l'un (5b) des implants - notamment de l'implant proximal (5b) - avec l'élément osseux (2) correspondant sont telles qu'elles autorisent son encastrement par insertion de cet implant (5b) selon une direction parallèle ou confondue à l'axe de cet élément osseux (2), et en ce que les surfaces de contact (21, 22) de l'autre (6b) implant - notamment de l'implant distal (6b) - avec l'élément osseux (3) correspondant sont telles qu'elles autorisent son encastrement par insertion de cet implant (6b) selon une direction transversale à l'axe de cet élément osseux (3).

6. Prothèse selon l'une des revendications 1 à 5 caractérisée en ce que l'implant proximal (5a, 5b) est constitué d'un bouclier proximal (9a, 9b) qui entoure l'extrémité (17, 19) de l'élément osseux (1, 2) encastré à l'intérieur d'une cavité (23a, 23b) formée par ce bouclier proximal (9a, 9b).

7. Prothèse métacarpophalangienne ou métatarsophalangienne selon la revendication 6 caractérisée en ce que le bouclier proximal (9a) définit une surface convexe (7a) qui présente une courbure autour d'un axe de courbure vertical - notamment en forme de portion de sphère ou de portion de tore - pour permettre notamment l'écartement des doigts en extension.

8. Prothèse selon l'une des revendications 6 et 7 caractérisée en ce que la cavité (23a) est en portion de sphère ou de tore.

9. Prothèse interphalangienne selon la revendication 6 caractérisée en ce que le bouclier proximal (9a) définit une surface convexe (7b) en forme de deux portions de sphères ou de tores accolées.

10. Prothèse selon l'une des revendications 6, 7 et 9 caractérisée en ce que la cavité (23a, 23b) est polyédrique.

11. Prothèse selon l'une des revendications 3 à 10 caractérisée en ce que le prolongement d'ancrage (10a, 10b) de l'implant proximal (5a, 5b) est une tige (10a, 10b) s'étendant axialement dans un perçage longitudinal de la partie médullaire (24, 25) de l'élément osseux (1, 2).

12. Prothèse selon la revendication 11 caractérisée en ce que la tige (10a) s'étend à partir de la surface interne du bouclier (9a) décalée vers le haut par rapport à l'axe médian longitudinal du bouclier (9a).

13. Prothèse interphalangienne selon l'une des revendications 1 à 12 caractérisée en ce que l'implant proximal (5b) est dépourvu de prolongement d'ancrage et est constitué d'un bouclier proximal (9b).

14. Prothèse selon l'une des revendications 1 à 13 caractérisée en ce que l'implant distal (6a, 6b) comporte un bouclier distal (11a, 11b) encastré dans un évidement ménagé dans l'extrémité (18, 20) de l'élément osseux (2, 3) qui entoure ce bouclier distal (11a, 11b).

15. Prothèse selon l'une des revendications 1 à 14 caractérisée en ce que le bouclier distal (11a, 11b) est globalement en forme de plaque transversale dont la surface de contact (21) avec l'élément osseux (2, 3) est plane et dont la surface libre forme la surface concave (8a, 8b) d'articulation.

16. Prothèse selon l'une des revendications 3 à 15 caractérisée en ce que le prolongement d'ancrage (12a, 12b) de l'implant distal (6a, 6b) est en forme d'aileron (12a, 12b) s'étendant dans un plan longitudinal médian de l'élément osseux (2, 3) au moins sensiblement perpendiculaire à l'axe principal (26a, 26b) de pivotement de l'articulation.

17. Prothèse selon la revendication 16 caractérisée en ce que la majeure partie de l'aileron (12a, 12b) s'étend dans une partie évidée de l'élément osseux (2, 3).

18. Prothèse selon l'une des revendications 16 et 17 caractérisée en ce que l'aileron (12a, 12b) est en forme de triangle rectangle dont un côté (29), adjacent au bouclier distal (11a, 11b), s'étend sur au moins une partie de la hauteur médiane du bouclier distal (11a, 11b).

19. Prothèse selon l'une des revendications 3 à 15 caractérisée en ce que le prolongement d'ancrage (12a, 12b) de l'implant distal (6a, 6b) est en forme de rail (12a, 12b) s'étendant dans un plan longitudinal médian de l'élément osseux (2, 3) au moins sensiblement perpendiculaire à l'axe principal (26a, 26b) de pivotement de l'articulation.

20. Prothèse selon la revendication 19 caractérisée en ce que la majeure partie du rail (12a, 12b) s'étend dans une partie évidée de l'élément osseux (2, 3).

21. Prothèse selon l'une des revendications 19 et 20 caractérisée en ce que le rail (12a, 12b) est en forme de rectangle dont un côté (29), adjacent au bouclier distal (11a, 11b), s'étend sur au moins une partie de la hauteur médiane du bouclier distal (11a, 11b).

22. Prothèse selon l'une des revendications 3 à 21 caractérisée en ce qu'au moins un prolongement d'ancrage (10a, 10b) comporte au moins un perçage transversal (34a, 34b, 36a, 36b) permettant sa fixation par une cheville ou une vis (35a, 35b, 37a, 37b) transversale traversant l'élément osseux (1, 2, 3).

23. Prothèse selon l'une des revendications 3 à 22 caractérisée en ce qu'au moins un prolongement d'ancrage (10a, 10b, 12a, 12b) comporte au moins un perçage transversal (38a, 38b, 40a, 40b) permettant la fixation d'un ligament naturel ou artificiel (13a, 13b) à ce prolongement d'ancrage (10a, 10b, 12a, 12b) notamment par une cheville ou une vis (39a, 39b) coinçant ce ligament (13a, 13b) dans le perçage transversal (38a, 38b, 40a, 40b).

24. Prothèse selon l'une des revendications 3 à 23 caractérisée en ce qu'au moins un prolongement d'ancrage (12a, 12b) comporte au moins un perçage transversal (41a, 41b) de fixation d'un tendon (14a, 14b).

25. Prothèse selon l'une des revendications 1 à 24 caractérisée en ce que le bouclier proximal (9a, 9b) est métallique , susceptible d'être réhabitable par l'élément osseux ou en céramique et en ce que le bouclier distal (11a, 11b) est, au moins pour sa portion formant la surface concave (8a, 8b) d'articulation, en matière synthétique - notamment en polyéthylène- ou en céramique.

26. Prothèse selon l'une des revendications 1 à 25 caractérisée en ce que l'implant proximal (5a, 5b) est constitué d'une pièce métallique monobloc, susceptible d'être réhabitable par l'élément osseux ou d'une pièce en céramique monobloc.

27. Prothèse selon l'une des revendications 1 à 26 caractérisée en ce que l'implant distal (6b) est constitué d'une pièce monobloc en matière synthétique - notamment en polyéthylène - ou en céramique.

28. Prothèse selon l'une des revendications 1 à 27 caractérisée en ce que l'implant distal (6a) est constitué de deux pièces associées rigidement l'une à l'autre : une pièce métallique ou en céramique (32) interne, susceptible d'être réhabitable par l'élément osseux, formant le prolongement d'ancrage (12a) et la partie interne du bouclier (9a) et une pièce de matière synthétique (33) - notamment en polyéthylène - ou en céramique formant la partie externe du bouclier (9a).

29. Prothèse selon l'une des revendications 1 à 28 caractérisée en ce qu'elle comporte un manchon (42) de prolongation artificiel interposé entre l'extrémité (17) d'au moins un os (1) et l'implant (5a) correspondant.

30. Prothèse selon la revendication 29 caractérisée en ce que le prolongement d'ancrage (10a) de l'implant (5a) traverse axialement tout le manchon (42) pour être associé rigidement à l'extrémité (17) de l'os (1).
